# EUROPÄISCHE PATENTSCHRIFT

(11) **EP 0 917 568 B1**
(45) Veröffentlichungstag und Bekanntmachung des Hinweises auf die Patenterteilung: **21.03.2007**
(21) Anmeldenummer: 96923851.8
(22) Anmeldetag: 16.07.1996
(51) Int. Cl.: C12N 9/96, C12Q 1/25, C12Q 1/48, C12Q 1/68, C12P 21/02, G01N 33/573

(54) **VERFAHREN ZUR HERSTELLUNG KOMPLEXER MULTIENZYMATISCHER LAGERSTABILER REAKTIONSGEMISCHE UND DEREN VERWENDUNG**
METHOD FOR PRODUCING COMPLEX MULTIENZYMATICAL, STORAGE RESISTANT REACTION MIXTURES AND USE THEREOF
PROCEDE DE PRODUCTION DE MELANGES REACTIONNELS MULTIENZYMATIQUES COMPLEXES, STABLES AU STOCKAGE, ET UTILISATION DE CES MELANGES

(43) Veröffentlichungstag der Anmeldung: 26.05.1999
(73) Patentinhaber: QIAGEN North American Holdings, Inc., Germantown, MD 20874 (US)
(72) Erfinder: PETERS, Lars-Erik, D-10367 Berlin (DE); BENDZKO, Peter, D-12623 Berlin (DE)
(74) Vertreter: Schneider, Jürgen M.
(86) Internationale Anmeldenummer: PCT/DE1996/001288
(87) Internationale Veröffentlichungsnummer: WO 1998/002532

(56) Entgegenhaltungen:
- EP-A- 0 365 685
- EP-A- 0 726 310
- DE-A- 19 503 685
- BIOTECHNOLOGY, Bd. 10, 10.September 1992, Seiten 1007-1011, XP000568746 COLACO ET AL.: "Extraordinary stability of enzymes dried in trehalose: simplified molecular biology."
- NUCLEIC ACIDS RESEARCH, Bd. 21, Nr. 12, 1993, Seiten 2959-2960, XP002027157 KAIJALAINEN S. ET AL. : "An alternative hot start technique for PCR in small volumes using beads of wax-embedded reaction components dried in trehalose."
- PROC. NATL. ACAD. SCI. USA, Bd. 91, Juni 1994, Seiten 5695-5699, XP002027158 CHENG S. ET AL. : "Effective amplification of long targets from cloned inserts and human genomic DNA."

## Beschreibung

Die Erfindung bezieht sich auf komplexe multienzymatische stabilisierte Reaktionsgemische aus nativen und gegebenenfalls artifiziellen enzymatisch aktiven Proteingemischen, die anwendungsfertig komplettiert sind und ohne Aktivitätsverlust bei Kühlschranktemperatur (0°- 10°C) gelagert und transportiert und für die Synthese, Modifizierung oder Analyse von Polypeptiden und Nukleinsäuren verwendet werden können.

Die Verwendung von komplexen Reaktionssystemen aus Zellextrakten oder Enzymgemischen zur Untersuchung biochemischer Reaktionsabläufe spielt eine immer wichtigere Rolle in der modernen Biologie und zunehmend auch in der medizinischen Diagnostik.

Probleme der Synthese, Faltung, der postranslationalen Reifung und des intrazellulären Targetings von Proteinen werden seit langem mit Hilfe von zellfreien Extrakten oder Lysaten untersucht, die den kompletten ribosomalen Apparat für die Proteinbiosynthese enthalten. Neben Anwendungen aus der Grundlagenforschung gewinnen multienzymatische Reaktionsgemische für die zellfreie Proteinbiosynthese (invitro Translation) zunehmend Bedeutung für die Lösung präparativ-synthetischer Zielstellungen. Die in-vitro Translation wurde ausserdem zur Synthese von Proteinteilstücken zur Kartierung von immunodominaten Epitopen, katalytischen Zentren oldersubstratbindungstellen eingesetzt. Seit der Einführung des sogenannten Protein-Truncation-Assay (PTU) zur Detektion von relevanten Genmutationen besteht ein wachsender Bedarf in der Tumordiagnostik an einfach zu handhabenen, standardisierten und anwendungfertigen Reaktionsgemischen für die *in-vitro* Translation von synthetischer, über RT-PCR hergestellte, mRNS.
Auch bei anderen analytisch-molekularbiologischen Methoden, wie der Polymerasekettenreaktion (PCR), der DNS-Sequenzierung und in-vitro RNS-Synthese *(in-vitro* Transkription) zeichnet sich ein Trend ab, klassische Einzelenzymassays durch multienztymatische Reaktionssysteme zu ersetzen. Die Kombination von mehreren Enzymen und Kofaktoren mit spezialisierten Funktionen erhöht die Prozessivität und veringert die Mutationsrate von DNS- und RNS-Polymerasen *in-vitro.* Im Ergebnis können wesentlich längere DNS-Fragmente (>10 kb) amplifiziert werden als mit nur einem Enzym. Die Synthesegenauigkeit und Produktausbeute sind höher, die Fehlsynthese unspezifischer Nebenprodukte wird besser unterdrückt. Zu den Enzymen und Proteinfaktoren, die in der PCR als multienzymatisches Reaktionssystem kombiniert werden, gehören inorganische Pyrophosphatase, DNS-Bindungsproteine, Polymerase-spezifische Antikörper und DNA Polymerasen mit verschiedenen Exonukleaseaktivitäten.
Beim gegenwärtigen Stand der Technik ist die Anwendung multienzymatischer Reaktionsgemische mit einer Reihe von Nachteilen bezüglich Handhabung, Reproduzierbarkeit und Lagerstabilität verbunden, welche die die Nutzung von multienzymatischen Reaktionsgemischen für die Protein- und DNS-Synthese in der angewandten Forschung und Diagnostik verzögern.

Der entscheidende Nachteil von multienzymatischen Reaktionssystemen gegenüber Einzelenzymassays besteht in der komlipizierten Handhabung und der eingeschränkten bzw. nicht vorhandenen Lagerstabilität. Komplette Reaktionsgemische mit allen Enzymen, Substraten und Kofaktoren sind als wäßrige Lösungen weder bei Raumtemperatur noch im gefrorenen Zustand über längere Zeit stabil. Das ergibt sich aus der Tatsache, daß die Bedingungen (pH-Wert, Ionenstärke, Konzentrationen der Enzyme und des Stabilisierungsmittels, Art der Salze), die optimal für die Durchführung der biochemischen Reaktion sind, von denen abweichen, die für die Konservierung und dauerhafte Stabilisierung der Enzymkomponenten und Kofaktoren notwendig sind (Franks, F. (1989) Process Biochem. 24 (1), R3-R7).
Bei multienzymatischen Reaktionsgemischen kommt das Problem hinzu, daß die einzelnen Komponenten der Zellextrakte oder Enzymgemische, je nachdem, ob es sich um lösliche Enzyme, fibrilläre Strukturproteine, membranassoziierte Enzymkomplexe oder Nukleoproteine handelt, unterschiedliche Anforderungen an das Stabilisierungsmedium und die Lagerbedingungen stellen. In den meisten Fällen lassen sich die verschiedenen Anforderungen nicht miteinander vereinbaren. Deshalb werden kommerzielle Reaktionssysteme für die in-vitro Translation, Transkription, PCR oder DNS-Sequenzierung in Form von Kits angeboten, in denen die Komponenten einzeln bereitgestellt werden. Wie das folgende Beispiel eines Reaktionsgemisches für die in-vitro Translation zeigt, müssen die verschiedenen Komponenten des Kits getrennt bei unterschiedlichen Bedingungen gelagert werden.

| **Komponenten des Reaktionsgemisches** | **Konzentrationsfaktor** | **Lagertemperatur** |
|---|---|---|
| 1. Mastermix (HEPES-KOH, ATP, GTP, DTT, tRNS, Speridin, Kreatinphosphat) | 12.5X | -20°C |
| 2. Aminosäuremix (2.5 mM von jeder Aminosäure) | 50X | -20°C |
| 3. Kreatinkinase | 25X | 4°C |
| 4. RNase Inhibitor | ----- | -20°C |
| 5. Zellextrakt/lysat (zellfreies Extrakt, K-Azetat, Mg-Azetat, HEPES-KOH, DTT) | 3X | -80°C |
| 6. Translationspuffer (K-Acetat/Mg-Azetat) | 25X | -20°C |

Dem Anwender obliegt es, jedesmal vor Versuchsbeginn den Reaktionsansatz aus den Einzelkomponenten zusammenzumischen. Dieser Arbeitsschritt ist fehleranfällig und läßt sich schwer automatisieren. Der Zeitaufwand potenziert sich mit der Anzahl der parallelen Reaktionen. Unter diesen Vorausetzungen nimmt die Versuchsvorbereitung oft mehr Zeit in Anspruch als die eigentliche Versuchsdurchführung.
Aufgrund der verschiedenen Temperaturanforderungen (4°C, -20°C und -80°C) ist der technische Aufwand für die Lagerung und Transport der Ausgangskomponenten entsprechend hoch. Die Versendung eines Kits für die *in-vitro* Translation muß in Trockeneis erfolgen, um ein Auftauen des Zellextraktes zu vermeiden.
Die empfindlichste und zugleich wichtigste Komponente von in-vitro Translationsassays ist das Zellextrakt mit den makromolekularen Nukleoproteinkomplexen für die ribosomale Proteinsynthese. Der komplexe biochemische Reaktionsablauf der RNS-gesteuerten Proteinsynthese erfordert die kooperative Wechselwirkung einer Vielzahl von Enzymen, Enzymkomplexen und Strukturproteinen mit unterschiedlicher Struktur und Stabilität. Anders als in den Zellen, wo die makromolaren Proteinkomplexe des ribosomalen Translationsapparates über die Interaktion mit den Filamenten des Zytoskellettes stabilisiert werden, enthalten die zellfreien Extrakte und Lysate kein intaktes Zytoskellett mehr. Frei in Lösung dissoziieren die makromolekularen Komplexe leicht und verlieren dadurch ihre Reaktionsfähigkeit.

Bisher ist die einzig zuverlässige Methode für die Konservierung von löslichen biologisch aktiven Zellextrakten die Lagerung im tiefgefrorenen Zustand bei -80°/-120°C. Damit sind eine Reihe von Problemen und Nachteilen verbunden. Beim Einfrieren verlieren Zellextrakte bzw. Lysate aus Weizenkeimen, Retikulozyten oder Bakterienzellen einen Teil ihrere ursprünglichen enzymatischen Aktivität, da durch die Bildung von Wasserkristallen viele Proteine irreversibel geschädigt werden. Mehrfaches Einfrieren und Auftauen für nachfolgende Versuche führt bei zellfreien Lysaten aus E. coli und Retikulozyten zum fast vollständigen Verlust der Translationaktivität. Bei Weizenkeimextrakten beträgt der Aktivitätsverlust etwa 20-40% nach jedem Auftauen und Einfrieren.

Die Instabilität von zellfreien Extrakten gegenüber wiederholtem Einfrieren und Auftauen zwingt den Anwender zu einem unwirtschaftlichen Verbrauch. Um reproduzierbare Versuchsergebnisse zu erzielen, darf jede Charge des translationsaktiven Zellextraktes nur einmal aufgetaut werden. Eine denkbare Alternative wäre die Lagerung kompletter anwendungsfertig zusammengestellter Reaktionsgemische im tiefgefrorenem Zustand bei -80°C. Tests mit Translationsassays auf der Basis von Weizenkeimextrakten zeigten, daß bereits ein einmaliges Einfrieren und einwöchige Lagerung bei -80°C zu einem Verlust von 60% der Translationsaktivität im Vergleich zur frisch angestzten Kontrollreaktion führt.
Konzentrierte zellfreie Proteinextrakte sind somit verhältnismäßig lagerstabil bei Tiefsttemperaturen, Reaktionsgemische mit entsprechend verdünnten Zellextrakten sind es nicht. Ein entscheidender Faktor dafür ist die hohe Proteinkonzentration in unverdünnten Zellextrakten, die stabilisierend wirkt.
Ähnlich verhält sich die Stabilitätsproblematik bei Reaktionsgemischen für die PCR, *in-vitro* Transkription und DNS-Sequenzierung. Das Einfrieren selbst in konzentrierten wäßrigen Lösungen deaktiviert DNS- und RNS-Polymerasen vollständig, so daß diese nur in Gegenwart hochkonzentrierter Kryoprotektoren lagerstabil bei -20°C sind. Das üblicherweise als Kryoprotektor verwendete Glycerin (50%) und andere (DMSO, Polyethylenglycol) beeinträchtigen in hohen Konzentrationen die PCR (Primer-Annealing) und in-vitro Transkription (Crowe, L. M. and J. H. Crowe, Dev. Biol. Stand. 74, 285-294)). Bei Glycerinkonzentrationen <50% sinkt die Lagerstabilität der Enzyme bei -20°C.
Seit dem Erscheinen der ersten Veröffentlichungen die Präparation zellfreier Extrakte für die in-vitro Translation Mitte der 70-iger Jahre hat sich wenig an der Methode zur Herstellung und stabilen Lagerung geändert. Eine Alternative zum beschriebenen Stand der Technik wäre die Lagerstabilmachung durch Gefriertrocknung. Diese Methode wurde erfolgreich zur Stabilisierung von Liposomen und Membranfraktionen, Einzelenzympräparaten oder von Teilreaktionsgemischen ohne Enzymkomponente angewendet, wobei spezielle Zucker oder Polyole in Kombination mit bivalenten Metallionen oder Tensiden die Denaturierung der Biomoleküle durch Wasserverlust verhinderten bzw. einschränkten.

Als besonders geeignet für die Lagerstabilmachung von Enzympräparaten erwies sich der Zucker Trehalose in Kombination mit bekannten Kryoprotektoren wie PEG oder DMSO (19,9,10). Dieser Zucker ist ein natürliches Stoffwechselprodukt vieler Pflanzen, Insekten und Mikroorganismen, das unter bestimmten Stressbedingungen (Hitzeschock, Dehydrierung, radioaktive Strahlung) intrazellulär angereichert wird und das Überleben dieser Organismen sichert.

Colaco et al. (BIOTECHNOLOGY, 10, 1992, Seiten 1007-1011) beschreiben die Verwendung von Trehalose zur Stabilisierung von Einzelenzymen während der Trocknung. Die Verwendung von Trehalose zur Stabilisierung von Enzymgemischen wird in der nachveröffentlichten EP 0726 310 angesprochen.Aufgabe der Erfindung ist, die Reaktionsfähigkeit, Lagerstabilität, den Transportsowie die anwendungsfertige Aufbereitung von komplexen multienzymatischen Reaktionsgemischen für die Durchführung biochemischer Reaktionsabläufe zu verbessern. Insbesondere soll das Verfahren zu Produkten führen, bei denen auf eine Tiefkühlung der zu lagernden und zu transportierenden Reaktionsgemische verzichtet werden kann, sowie durch die Bereitstellung anwendungsfertiger, d.h. mit allen Reaktionskomponenten versehenden, Reaktionsgemische den experimentellen Aufwand für den Anwender erheblich reduzieren und die Reproduzierbarkeit erhöhen.

Die Aufgabe wird erfindungsgemäss durch die in Anspruch 1 angegebene Verwendung von 8-12% Trehalose zur Stabilisierung und zur Steigerung der Reaktionsfähigkeit von multienzymatischen Reaktionsgemischen, die gefriergetrocknet werden, gelöst. Die Unteransprüche betreffen spezielle Ausführungsformen.

Die erfindungsgemässe Verwendung beruht auf dem überrasschenden Effekt dass native oder artifizielle enzymatisch aktive Proteingemische mit den Reaktionskomponenten und Trehalose in wässriger Lösung miteinander kombiniert werden können, wobei einerseits die Reaktionsfähigkeit des multienzymatischen Systems erhöht und andererseits die instabilen Reaktionskomponenten vor dem Verlust ihrer biologischen Aktivität bzw. ihrer biologisch aktiven Struktur während der Lagerstabilmachung und Lagerung geschützt werden, wenn sie anschliessend durch Gefriertrocknung in einen bei0°-10°C lagerstabilen Zustand überführt werden, und wobei die Menge des Stabilisierungsmittels, die zur Erhöhung der enzymatischen Aktivität des Reaktionsgemisches führt, equivalent zu der Menge ist, die für die Lagerstabilmachung des komplexen multienzymatischen Reaktionsgemisches benötigt wird.

Als native enzymatisch akive Proteingemische Zellextrakte, Zellysate oder Fraktionen aus diesen eingesetzt.

Als artifizielle enzymatisch aktive Proteingemische wird eine Kombination aus vorgereinigten Einzelenzymen, Cofaktoren und gegegenfalls Strukturproteinen eingesetzt die gegebenenfalls verschiedener Herkunft sind.

Reaktionskomponenten gemäss der Erfindung sind enzymatische und nichtenzymatische Kofaktoren, Enzymsubstrate, Nukleotide und Nukleoside oder deren Oligomere, Proteiene, Preptide, Thiolverbindungen, RNS, DNS und gegebenfalls Derivate jeder der obigen Substanzen einzeln oder in Kombination.

Zur Stabilisierung wird erfindungsgemäß Trehalose verwendet, der bei einer Konzentration von8-12% (M/Vol) in wässriger Lösung im anwendungsfertigen Reaktionsgemisch optimale Bedingungen für die Stabilisierung der labilen Reaktionskomponenten erzeugt und ausserdem die maximale spezifische Produktausbeute der multienzymatischen Reaktionsgeische während der Synthese gewährleistet.

Gegebenenfalls wird eine Vakuumtrocknung der multienzymatischen Reaktionsgemische bei Raumtemperatur in einer handelsüblichen Lyophilisationsanlage in einer Zeitspanne von 3-4 Stunden durchgeführt, wobei die Reaktionsgemische gegebenenfalls unmittelbar vor der Vakuumtrocknung in flüssigem Stickstoff oder gegebenfalls in einem Trockeneis/Alkoholbad eingefroren werden.

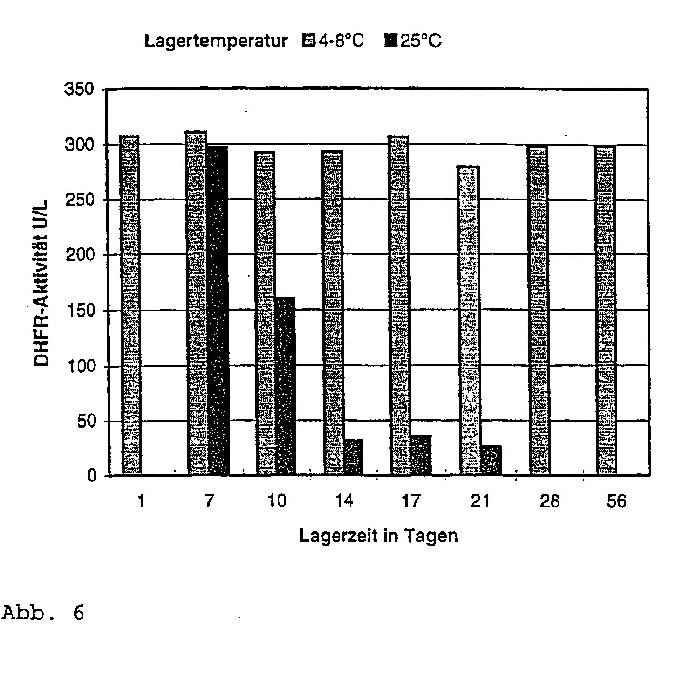

Die Erfindungbetrifft bevorzugt multienzymatische Reaktionsgemische für die in-vitro Translation in Weizenkeimextrakten und PCR.50ul- Reaktionsgemische wurden nach dem erfindungsgemässen Vefahren (Ausführungsbeispiel 1) angesetzt und gefriergetrocknet. Nach verschiedenen Lagerfristen wurden die getrockneten Reaktionsgemische in Wasser unter Zugabe der entsprechenden mRNA und gegebenfalls einer radioaktiv markierten Aminosäure rekonstituiert. Die Reaktionsfähigkeit der so behandelten und gelagerten Reaktionsgemische wurde mittels Translation verschiedener mRNS nachgewiesen: Dehydofolatreduktase (DHFR, 17.5 kD) und Obelin (20 kD). Der quantitative Nachweis des Translationsproduktes erfolgte über die Messung des TCAgefällten radioaktiv markierten Proteins ausSul des Reaktionsansatzes, oder durch Bestimmung der enzymatischen Aktivität der DHFR in 10 ul des Reaktionsansatzes nach 2 Stunden Inkubation bei 250C. Der qualitative Nachweis des Translationsproduktes erfolgte mittels Gelelektrophorese im SDS-PAG und anschliessender Radioautographie. Die Translationsausbeute in den rekonstituierten gefriergetrockneten Reaktionsgemischen wurde mit jeweils mit der Produktausbeute in unbehandelten Reaktionsansätzen mit und ohne Trehalose verglichen, um die Effektivität der Lagerstabilmachung zu bestimmen. Die Translationaktivität der rekonstituierten Reaktionsgemische nach 1-3 Monate Lagerung bei 40C schwankte zwischen 92-1000C im Vergleich zur Aktivität in den den unbehandelten Kontrollreaktion mit 10% Trehalose (Abb. 6).

Die Reaktionsfähigkeit rekonstituierter gefriergetrockneter PCR-Reaktionsgemische mit einem artifiziellen Enzymgemisch wurde in einem RAPD-PCR-Assay überprüft. Das artifizielleEnzymgemisch für die PCR bestand aus der- Taq-DNS-Polymerase, derDeep-Vent-DNS-Polymerase und der inorganischen Tth Pyrophosphatase im Mischungsverhältnis 10:1:0,2 (Einheiten).

Die erfindungsgemässe Verwendung ermöglicht so die anwendungsfertige Aufbereitung von nativen und artifiziellen enzymatisch aktiven Proteingemischen mit Reaktionspuffern, Kofaktoren und Substraten in dem den Reaktionsgemischen in wässriger Lösung
Trehalose zugeführt wird, das einerseits die
Reaktionsfähigkeit des multienzymatischen Systems verbessert und andererseits die labilen Reaktionskomponenten vor dem
Verlust ihrer Aktivität bzw. ihr biologisch aktiven Struktur während der Lagerstabilmachung schützt, diese nach Einfrieren in flüssigem Stickstoff vakuumgetrocknet werden, ggf. eine Überschichtung mit inertem Gas erfolgt.

Die so erhaltenen lagerstabilen Reaktionsgemische werden nach Rekonstituierung in Wasser (Milli-Q Qualität) durch Zuführung von anwenderspezifischen Schlüsselkomponenten je nach gewünschter enzymatischer Reaktion für die Synthese, Modifikation oder Ananlyse von Proteinen, Polypetiden oder Nukleinsäuren verwendet.

Die erfindungsgemäss hergestellten Reaktionsgemische haben den Vorteil, dass sie bei 0°-10°C stabil gelagert und transportiert werden können. Dadurch entfällt der technisch hohe Aufwand für Anschaffung und Unterhaltung einer Tiefkühlanlage, wie sie nach herkömmlichen Stand der Technik notwendig ist. Ein zweiter Vorteil besteht darin, daß die Reaktionsgemische anwendungsfertig mit allen notwendigen Komponenten aufbereitet sind, so daß der Anwender die gewünschte Reaktion nur durch die Zugabe einer, oder maximal zweier Schlüsselkomponenten starten kann. Das hebt die Nachteile des Standes der Technik auf bezüglich:
- der simultanen Durchführung einer großen Zahl von Parallelversuchen (Epitopkartierung),
- der Reproduzierbartkeit von parallelen und aufeinanderfolgenden Enzymreaktionen
- des minimalen Zeitaufwandes für die Versuchsvorbereitung,
- der Fehleranfälligkeit des Vorbereitens komplexer Reaktionsgemische
- der Automatisierung komplexer biochemischer Reaktionsabläufe im analytischen Maßstab.

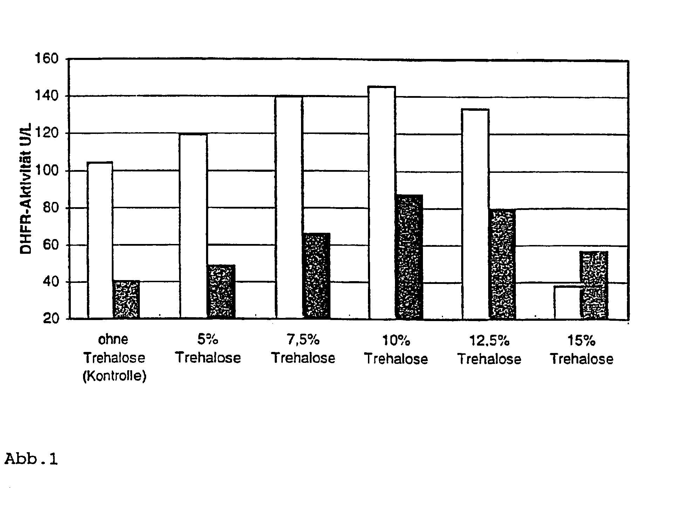

Die vorliegende Erfindung basiert auf der Entdeckung, daß Trehalose neben seiner bekannten Schutzwirkung bei Dehydrierung die spezifische Produktausbeute in der *in-vitro* Translation mit Weizemkeimextrakten und PCR erhöht. In unbehandelten, d.h. frisch angesetzten, Translationsreaktionen steigt die spezifische Produktausbeute (synthetisiertes Protein pro eingesetzte mRNA) im Vergleich zu Reaktionsansätzen ohne Trehalose in Abhängigkeit von der Trehalosekonzentration. Die maximale Produkausbeute wird bei 10% w/v erzielt (Abb. 1).

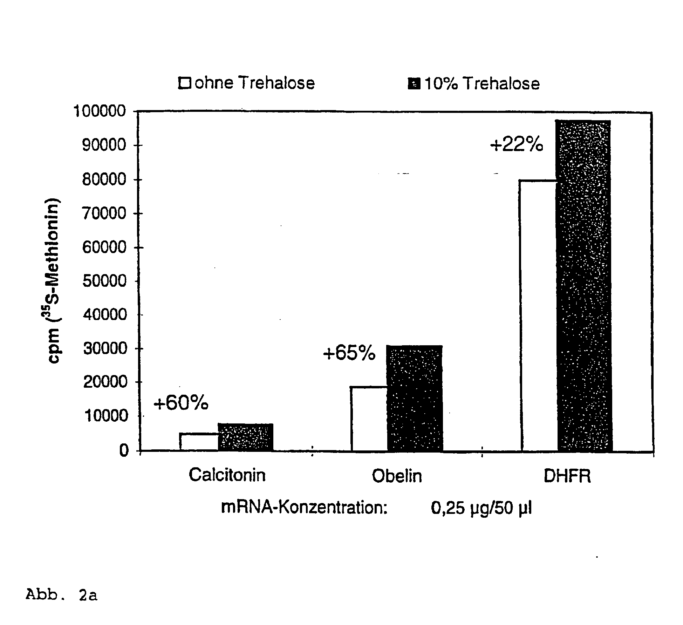

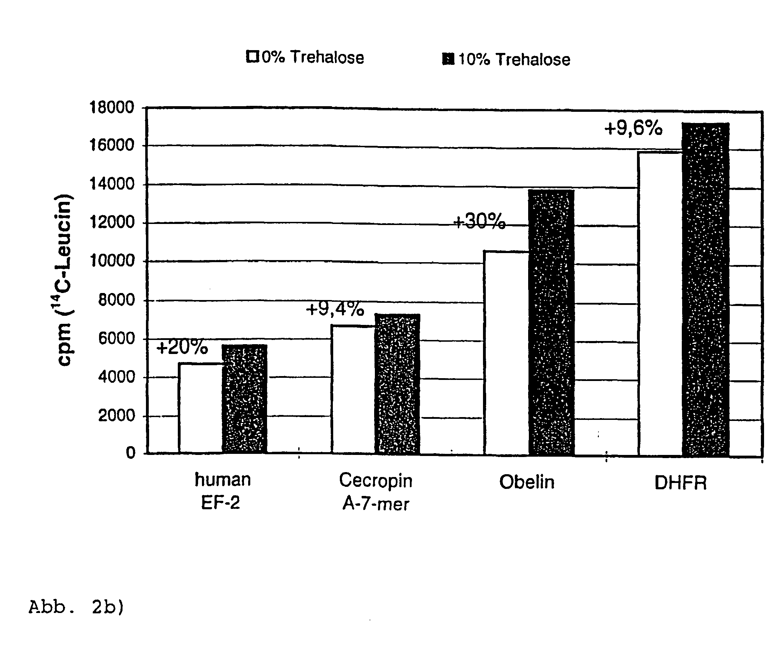

Der 'Enhancer'-Effekt der Trehalose in wäßriger Lösung konnte für eine Reihe von Modellproteinen und verschiedenen Weizenkeimextrakten validiert werden (Abb. 2a und 2b), so daß es sich um ein produktunabhängiges universielles Phänomen handelt.

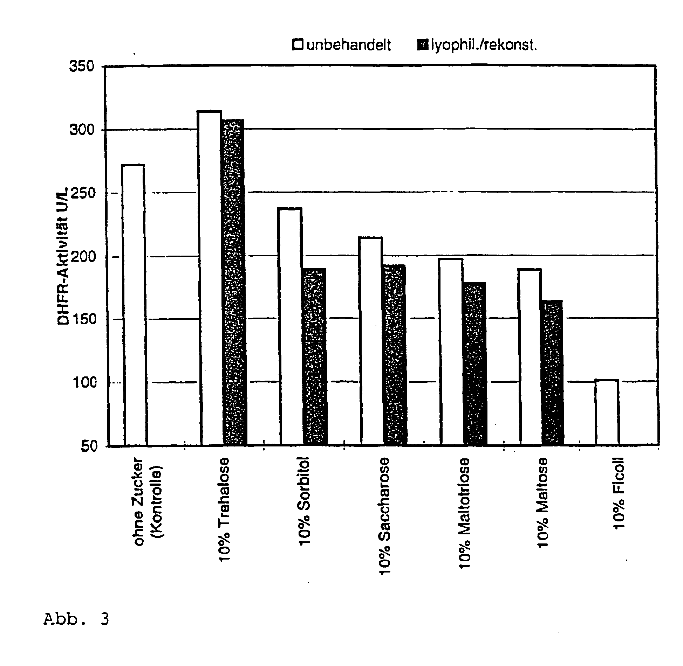

Die neu entdeckte Wirkung ist eine unikale Eigenschaft der Trehalose. Alle anderen untersuchten Hilfsstoffe, die nach dem Stand der Technik ebenso wie Trehalose als effektive Kryoprotktoren bzw. Stabilisatoren für die Vakuumtrockung verwendet werden, inhibieren die *in-vitro* Translation im Konzentrationsbereich, der für die Lagerstabilmachung notwendig ist (Abb. 3).

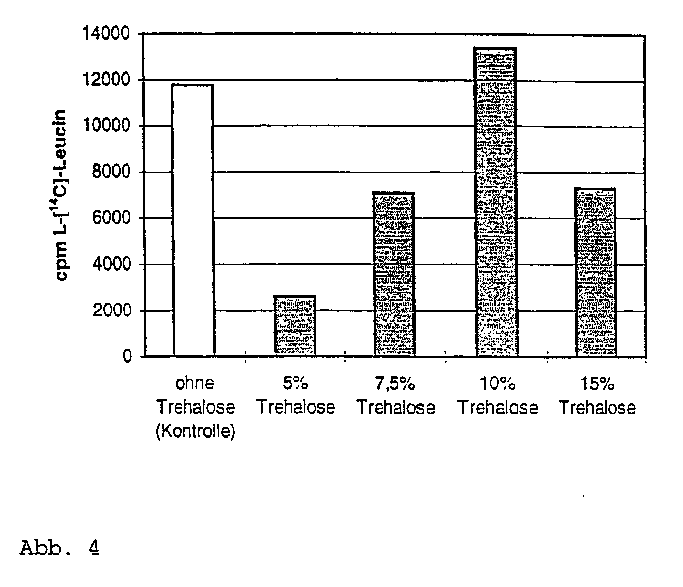

Es stellte sich außerdem heraus, daß die optimale Trehalosekonzentration für die Lagerstabilmachung durch Gefriertrockung dem Konzentrationsoptimum in der Translationsreaktion in wäßriger Lösung entspricht (Abb. 4).

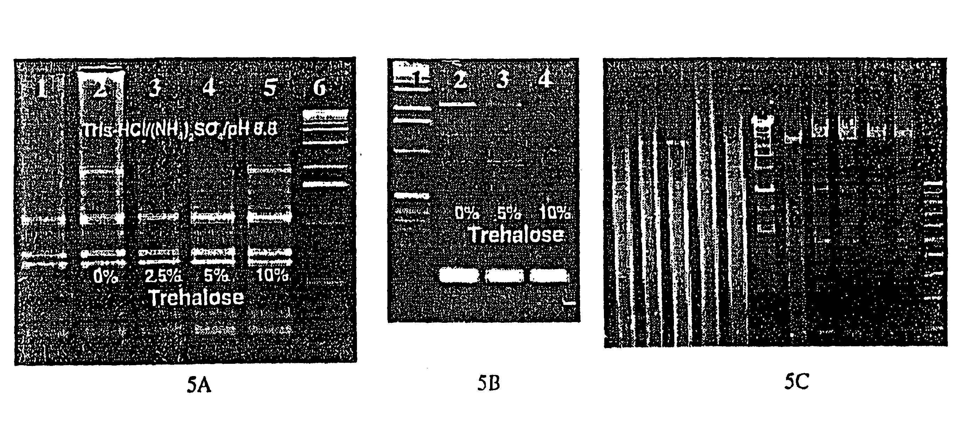

Auch in PCR-Anwendungen wurde ein 'Enhancer'-Effekt der Trehalose entdeckt. Ähnlich wie bei der in.vitro Translation erhöht sich die Ausbeute des (der) spezifischen Amplifikate mit steigender Trehalosekonzentration, während die Amplifikation unspezifischer DNS-Fragmente unterdrückt wird (Abb. 5a/5b).

Besonders drastisch ist der Trehalose-Effekt in wäßriger Lösung bei der Amplifikation von DNS-Fragmenten > 10kb in Tris-HCl-Reaktionspuffern, wo ohne Trehalose kein spezifisches Produkt amplifiziert wird (Abb. 5c).

Demnach unterscheidet sich die vorliegende Erfindung vom Stand der Technik zur Verwendung von Trehalose als Stabilisator in zwei wesentlichen Punkten. Erstens, können mit dem erfindungsgemäßen Verfahren überraschend komplette, multienzymatische und anwendungsfertige Reaktionsgemische mit meheren labilen Proteinkomponenten und nicht Einzelenzyme bzw. Teilreaktionsgemische ohne Enzym lagerstabil und ohne Aktivitätsverlust hergestellt werden. Zweitens, sind unter Anwendung des neu entdeckten 'Enhancer'-Effektes der Trehalose die Bedingungen im erfindungsgemäßen Verfahren so optimiert, daß Trehalose als einziger Hilfsstoff eine ausreichende (Langzeit-) Lagerstabilität gewährleistet und gleichzeitig die Aktivität der rekonstituierten multienzymatischen Reaktionsgmische erhöht. Im Ergebnis der Anwendung des erfindungsgemäßen Verfahrens ist die Reaktionsfähigkeit rekonstituierter Reaktionsgemische höher als bei bei unbehandelten, 'frischen' Reaktionsansätzen ohne Stabilisator. Drittens, die lyophilisierten Reaktionsgemische sind im Gegensatz zu bekannten Verfahren nur im Temperaturbereich zwischen 0°-10°C lagerstabil für mindestens 6 Monate ohne Aktivitätsverlust. Eine Lagerung bei Temperaturen >15°C und <0°C führt zum vollständigen Aktivitätsverluast innerhalb 1 Monats.

Die Erfindung soll nachstehend an zwei Beispielen erläutert werden.

### 1. Ausführungsbeispiel:

### Herstellung eines lagerstabilen translationsaktiven Reaktionsgemisches auf der Basis von weizenkeimextrakt unter Verwendung des Stabilisators Trehalose

a) **Herstellung des anwendungsfertigen Reaktionsgemisches aus den Einzelkomponenten**
Das Reaktionsgemisch wird auf Eis (0°-4°C) in einem sterilen 2.0ml-Mikrozentrifugengefäß (Schraubdeckel mit Gummidichtung und flacher Boden sind wichtig !) aus folgenden Einzelkomponenten zusammenpipettiert:

| **Reih enfolge** | **Komponenten und Zusammensetzung** | **Volumina** |
|---|---|---|
| **1.** | **milliQ-H₂O** | 13 µl |
| **2.** | **Aminosäuremix** (2.5 mM von jeder der 20 Aminosäuren) | 2 µl |
| **3.** | **Mastermix** (312 mM HEPES-KOH pH 7.6, 12.5mM ATP, 1.25mM GTP, 100mM Kreatinphosphat, 625µg/ml yeast tRNA, 3.125mM Spermidin, 25mM DTT) | 4 µl |
| **4.** | **Kreatinphospbokinase** 1.4 mg/ml | 2 µl |
| **5.** | 1M **Kalium-Acetat** | 2µl |
| **6.** | 25mM **Mg-Acetat** | 1µl |
| **7.** | 50% **Trehalose** | 10 µl |
| **8.** | **Weizenkeimextrakt** (90-100 OD₂₆₀) | 16 µl |

Die Komponenten im Reaktionsgefäß vorsichtig durchmischen (nicht vortexen). Das Volumen des fertigen Reaktionsansatzes vor der Lagerstabilmachung beträgt 50 µl. Für 25- oder 100µl-Ansätze müssen die Volumina der Einzelkomponenten porportional geändert werden.
b) **Lagerstabilmachung der Translationsgemische durch Gefriertrocknung (Lyophilisation)**
Die offenen 2.0 ml Reaktionsgefäße mit den Translationsgemischen werden unmittelbar nach der Durchmischung in flüssigen Stickstoff schockgefroren (-120°C) und für 5 Minuten im flüssigen Stickststoff inkubiert. Danach werden die gefrorenen Reaktionsgemische so schnell wie möglich in eine Lyophilisationskammer überführt, die an eine handelsübliche Vakuum-Ölpumpe angeschlossen ist. Die Vakuumpumpe muß bereits 30-60 Minuten Vorlauf haben, damit sofort nach Öffnen des Ventils ein starkes Vakuum im der Lyophilisationskammer aufgebaut wird. Im Labor der Erfinder wurde eine Lyophilisationsanlage von Heto-Lab benutzt. Die Reaktionsgemische werden 3-4 Stunden bei Raumtemperatur (20-30°C) lyophilisiert. Nach Beendigung der Lyophilisation wird die Vakuumkammer vorsichtig mit der Umgebungsluft oder gegebenenfalls mit einem inerten Gas belüftet. Der Lufteinlaß ist mit einem Sterilfilter zu versehen, um eine mikrobielle Kontamination der lyophilisierten Reaktionsgemische zu verhindern. Danach werden die Reaktionsgefäße unter sterilen Bedingungen luftdicht mit den Schraubdeckeln verschlossen und zusätzlich mit Parafilm versiegelt. Gegebenfalls können als Gefäße für die Gefriertrocknung auch Glasampullen verwendet werden, die dann entsprechend zugeschmolzen werden.
In diesem Zustand werden die lyophilisierten Reaktionsgemische lichtgeschützt im Kühlschrank bei 0-4°C gelagert.
c) **Rekonstituierung und *in-vitro* Translation**
Die lyophilisierten Reaktionsgemische werden auf Eis gestellt und in 48 µl Milli-Q-H₂O aufgelöst. Die getrockneten Rückstände lösen sich sofort in Wasser auf. Danach erfolgt die Zufgabe von 2 µl mRNS-Lösung (0.5-2µg/µl). Durch vorsichtiges Auf- und Abpipettieren wird der Reaktionsansatz durchmischt. Der rekonstituierte Reaktionsansatz wird für die *in-vitro* Translation für 2-3 Stunden bei 25°C inkubiert. Im Falle einer radioaktiven Markierung des Translationproduktes mit L-[¹⁴C]-Leucin (oder ³⁵S-Methionin) erfolgt di Rekonstituierung in 44µl Milli-Q-H₂O, 2 µl mRNA und 4µl Leucin-Lösung. Nach der angebenen Inkubationszeit wird die Menge des Translationproduktes in 5 bzw. 10µl des Reaktionsansatzes bestimmt. Die Bestimmung erfolgt entweder enzymatisch (DHFR-Enzymaktivität) oder über die Messung der säurefällbaren Readioaktivität (in cpm) im Scintillationsmeßgerät nach Standardprozeduren.

### 2. Ausführungsbeispiel:

### Herstellung eines lagerstabilen Reaktionsgemisches für Long-Range und RAPD-PCR auf der Basis eines Enzymmixes aus Taq-DNA-Polymerase, Pfu-DNA-Polymerase und inorganischer Pyrophosphatase von Thermus thermophilus

a) **Herstellung des anwendungsfertigen Reaktionsgemisches aus den Einzelkomponenten**
Das Reaktionsgemisch wird auf Eis (0°-4°C) in einem sterilen 0.5 ml-passend für den entsprechenden Thermocycler) aus folgenden Einzelkomponenten zusammenpipettiert :

| **Reih enfolge** | **Komponenten und Zusammensetzung** | **Volumina** |
|---|---|---|
| **1.** | **milliQ-H₂O** | 30 µl |
| **2.** | **10X Reaktionspuffer** (500mM Tricine-KOH pH 9.2, 160mM (NH₄)₂SO₄, 0.1% Tween 20) | 5µl |
| **3.** | **50X dNTP-Mix** (12.5mM dATP, dGTP, dCTP, dTTP) | 1 µl |
| **4.** | 50 mM **MgCl₂** | 1.5 µl |
| **5.** | Forward Primer (10 pmol/µl) | 1µl |
| **6.** | Reverse Primer (10 pmol/µl) | 1µl |
| **7.** | 50% **Trehalose** | 10 µl |
| **8.** | **Gelatine** (20mg/ml) | 0.5 µl |

Die Komponenten im Reaktionsgefäß durchmischen und abzentrifugieren. Das Volumen des fertigen Reaktionsansatzes vor der Lagerstabilmachung beträgt 50 µl. Die Schlüsselkomponente für den Start der PCR ist die entsprechende Template-DNS. Für 25- oder 100µl-Ansätze müssen die Volumina der Einzelkomponenten porportional geändert werden.
b) **Lagerstabilmachung der PCR-Gemische durch Gefriertrocknung (Lyophilisation)**
Entsprechend Ausführungsbeispiel 1b).
c) **Rekonstituierung und PCR**
Die lyophilisierten Reaktionsgemische werden auf Eis gestellt und in 48 µl Milli-Q-H₂O aufgelöst. Die getrockneten Rückstände lösen sich sofort in Wasser auf. Danach erfolgt die Zufgabe von 2 µl Template-DNS-Lösung (5-50ng/µl). Durch vorsichtiges Auf- und Abpipettieren wird der Reaktionsansatz durchmischt. Der rekonstituierte Reaktionsansatz wird direkt vom Eisbad auf ein vorgeheizten Thermocycler (94°C) überführt. Es folgt ein 2-4 minütiger Denaturierungschritt, dann wird je nach Anwendung eines der beiden PCR-Programme gestartet:

| | | | | |
|---|---|---|---|---|
| RAPD-PCR: 94°C | | 20 sek. | Long-Range PCR: 94°C | |
| 10 Sek. | | | | |
| 37°C | 30 Sek. | | 65°C | 20 Sek. |
| 72°C | 60 Sek. | | 68°C | 10 Min. |
| 35 Zyklen | | | | 25 Zyklen |

1. Jeweils 10µl des Reaktionsgemisches werden nach Beendigung des Programmes auf 0.8% TAE-Agarosegel aufgetragen und elektrophoretisch analysiert.

### Legende zu den Abbildungen

### Abbildung 1. DHFR-Syntheseausbeute in unbehandelten Reaktionsgemischen bei verschiedenen Trehalosekonzentrationen zum Zeitpunkt der maximalen Produktakkumulation (2 h), getestet mit mit verschiedenen Weizenkeimextrakten.

a) Auf der Basis von hochaktivem Weizenkeimextrakt (Lysat SL, weiße Balken) und zellfreiem Extrakt aus weniger aktiven Weizenkeimen (Lysat JB, schwarze Balken) wurden 2 Serien von DHFR-Translationsreaktionen (50µl, 2µg DHFR-mRNA) angesetzt mit steigenden Trehalosekonzentrationen. Nach 2 Stunden Synthesedauer der *in-vitro* Translation bei 25°C wurde die maximale Produktkonzentration im Reaktionansatz erreicht (siehe Translationkinetiken in Abb. 6a). Die Produktausbeute (DHFR-Aktivität) steigt mit wachsender Trehalosekonzentration und erreicht bei 10% (M/Vol) sein Maximum. Für beide Weizenkeimextrakte liegt die optimale Trehalosekonzentration bei 10% trotz verschiedener Translationsakitivität. Beim schwächer aktiven Lysat fällt eine Verschiebung der Konzentrationsabhängigkeit zugunsten höherer Trehalosekonzentrationen (12,5% und 15%) auf.

### Abbildung 2a. 'Enhancer'-Effekt der Trehalose bei der in-vitro Translation verschiedener mRNA's in unbehandelten Reaktionsgemischen.

Bestimmung der Produktausbeute (säurefällbare Radioaktivität) in 50µl-Translationsreaktionen mit 3 ausgewählten mRNA's mit und ohne Trehalosezusatz. Zur radioaktiven Markierung des Translationsproduktes wurden pro Reaktion jeweils 1µl [³⁵S]-Methionin (15 pmol = 349.440 cpm) eingesetzt. Zu Messung des säurefallbaren markierten Translationproduktes wurden nach 2 Stunden dem Reaktionsgemisch 2µl entnommen. Folgende Modellproteine wurden untersucht:
- humanes Calcitonin (120 bp, 0.25µg),
- Obelin (700 bp, 0.25µg),
- *E.coli* DHFR (500 bp, 0.25µg).
Die Translationsreaktionen mit [³⁵S]-Methionin-Markierung wurden bei einer unterkritischen RNA-Konzentration durchgegührt. Bei höhreren RNA-Konzentrationen erreichte die Synthese bereits nach 15 Minuten Inkubation ihren Sättigungspunkt aufgrund der limitierenden Methioninkonzentration im Reaktionsansatz.

### Abbildung 2b. Demonstration des positiven Trehaloseeffektes in unbehandelten radioaktiven Translationsassays mit [¹⁴C]-Leucin-Markierung verschiedener Modellproteine.

Vergleich der Produktausbeute (säurefällbare Radioaktivität) in 50µl-Translationsreaktionen mit und ohne Trehalose. Zur radioaktiven Markierung des Translationsproduktes wurden pro Reaktion jeweils 4µl [¹⁴C]-Leucin (624 pmol = 349.440 cpm) eingesetzt. Zu Messung des säurefallbaren markierten Translationproduktes wurden nach 3 Stunden dem Reaktionsgemisch 5µl entnommen. Folgende Modellproteine wurden verglichen: humaner Elongationsfaktor 2 (hEF 2, 300 bp, 2.0µg RNA), ein Oligomerkonstrukt des antibakteriellen Peptids Cecropin A (Cecropin A-7-mer, 2.5µg RNA), Obelin (700 bp, 2.0µg RNA), *E.coli* DHFR (500 bp, 1.5µg RNA).

### Abbildung 3. Vergleich der Wirkung bekannter Stabilisatoren auf die DHFR in-vitro Translation in unbehandelten und rekonstituierten gefriergetrockneten Reaktionsgemischen.

Zwei Serien von DHFR-Translationsreaktionen wurden unter Standardbedingungen (50µl, 2µg DHFR-mRNA, 2 h, 25°C) durchgeführt mit jeweils 10% m/vol Enkonzentration der ausgewählten Zucker. Die erste Serie (weiße Balken) von Translationsreaktionen bestand aus unbehandelten Reaktionsgemischen, die unmittelbar vor dem Start der in-vitro Synthese aus den Einzelkomponenten zusammengemixt wurden. Für die zweite Versuchsserie (schwarze Balken) wurden komplette Reaktionsgemische hergestellt, die zunächst gefriergetrocknet wurden und dann für die Synthese in 48µl Bidest Wasser rekonstituiert wurden. Zur Kontrolle für den Vergleich der Translationsausbeuten (DHFR-Aktivität) wurde eine DHFR-Translationsreaktion in einem Standardreaktionsgemisch ohne Zuckerzusatz durchgeführt. Die DHFR-Aktivität wurde aus jeweils 10µl eines Reaktionssansatzes bestimmt.

### Abbildung 4. Vergleich der DHFR-Syntheseausbeute in rekonstituierten Translationsreaktionsgemischen mit verschiedene Trehalosekonzentrationen. Bestimmung der optimalen Trehalosekonzentration für die Lagerstabilmachung.

Nach 3 Stunden Synthesedauer bei 25°C wurde die maximale Menge des säurefällbaren radioaktiv-markierten Translationsproduktes erreicht (siehe Translationkinetiken in Abb. 3). Wie bei dem vorangegangenen Experiment konnte die höchste Produktausbeute bei 10% (M/Vol) Trehalose erreicht. Eine höhere Konzentration (15%) inhibiert die Translation. Die Reduktion der Translationsausbeute bei Trehalosekonzentrationen <10% im Vergleich zur Kontrollreaktion (unbehandeltes Translationsgemisch ohne Trehalose, nicht gefriergetrocknet) ist eher auf die ungenügende Stabilisierung des translationsaktiven Weizenkeimlysats während der Gefriertrocknung zurückzuführen.

### Abbildung 5a: Einfluß der Trehalose auf die Performance von RAPD-PCR-Assays

RAPD-PCR mit einem 10-mer Zufallsprimer und Insekten-DNA in unbehandelten Reaktionsgemischen mit verschiednen Trehalosekonzentrationen. Die RAPD PCR wurde unter Standardbedingungen mit 250 ng genomischer DNA aus *Aeshna cynea* durchgeführt. Die Zugabe von Trehalose reduziert den unspezifischen Hintergrund, erzeugt durch Falschamplifikate, und verstärkt mit zunehmender Konzentration die Amplifikaion der längeren polymorphen DNA-Fragmente (> 2kb).
*Spur 1*: RAPD-PCR im Standard-PCR-Puffer [50mM Tris-HCl (pH 8.3 at 25°C)-50mM KCl-0.1% Triton 100].
*Spur 2*: RAPD-PCR im Tris--PCR-Puffer II [50mM Tris-HCl (pH 8.8 at 25°C)-16mM (NH₄)₂SO₄-0.01% Tween 20].
*Spur 3*-*5*: RAPD-PCR im Tris-PCR-Puffer II angereichert mit 2.5%, 5%, und 10% v/w Trehalose.
*Spur 6*: DNA 1 Kb Ladder.

### Abbildung 5b: Trehaloseeffekt in unbehandelten PCR-Reaktionsgemischen

Amplification eines 98 bp Fragmentes aus dem Cyctischen Fibrosisgene unter Standardbedingungen. Die Zugabe von Trehalose in unbehandelte Reaktionsansätze reduziert konzentrationsabhängig den unspezifischen Hintergrund (falsche Amplifikate bis 2kb).
*Spur 1* : DNA 1 Kb Ladder.
*Spur 2*: PCR im Standardreaktionsgmisch ohne Trehalose.
*Spur 3*: PCR in der Gegenwart von 5% Trehalose,
*Spur 4*: PCR in der Gegenwart von 10% Trehalose.

### Abbildung 5c: Effekt der Trehalose auf die Long-Range-PCR in Tris-HCl-Reaktionspuffern.

Long Range-PCR von einem 20 kb 1DNA-Fragment in unbehandelten Reaktionsgemsichen mit verschiedenen Tris-HCl Reaktionspuffern mit und ohne Trehalose unter Verwendung eines artifiziellen Enzymgemisches (Taq-DNA-Polymerase, Pfu-DNA-Polymerase, inorg. Pyrophosphatase). Die Zugabe von 10% w/v Trehalose ist notwendige und hinreichende Bedingung für die Amplifizierung des spezifischen DNA-Fragmentes unter den beschriebenen Bedingungen.
*Spur 1* : LR-PCR im Standard-PCR-Puffer(Tris-HCl/KCl/Triton X100 pH 8.3).
*Spur 2*: LR-PCR in Tris-HCl/(NH₄)₂SO₄/Tween 20 (pH 8.8).
*Spuren 3*-*5*: LR-PCR in kommerziellen Tris-HCl-Reaktionspuffern.
*Spur 6*: High molecular weight DNA-Marker 9-48 kb (GIBCO BRL).
*Spuren 7*-*11*: Die gleichen Reaktionsansätze wie in Spur 1-5, aber mit 10% Trehalose.
*Spur 12:* DNA 1Kb Ladder (GIBCO BRL).

### Abbildung 6. Versuche zur Langzeitstabilität von gefriergetrockneten Translationsassays.

50µl-Translationsreaktionsgemische, komplett mit allen Komponenten bis auf die DHFR-mRNA wurden nach den bekannten Bedingungen gefriergetrocknet und lichtgeschützt bei zwei verschiedenen Temperaturen gelagert. Nach verschiedenen Zeitabständen wurden Translationsreaktionen unter den Standardbedingungen durchgeführt (50µl, 2µg DHFR-mRNA, 2 h, 25°C) und die DHFR-Aktivität bestimmt. Für die Translationreaktionen wurde immer die gleiche mRNA-Präparation verwendet, um ausschließlich die Abhängigkeit der Translationsaktivität der lyophilisierten Reaktiongemische von der Lagertemperatur und Zeit zu bestimmen.

## Patentansprüche

1. Verwendung von Trehalose in multienzymatischen Reaktionsgemischen, die durch Gefriertrocknung in einen lagerstabilen Zustand überführt werden zur gleichzeitigen Erhöhung der Lagerstabilität und der Reaktionsfähigkeit dergestalt, dass nach Gefriertrocknung rekonstituierte Reaktionsgemische verglichen mit frischen Reaktionsgemischen ohne Trehalose eine höhere enzymatische Aktivität aufweisen, wobei die Reaktionsgemische native oder artifizielle aktive Proteingemische enhalten und die Trehalose in einer Konzentration von 8-12% (Masse/Vol) dem Reaktionsgemisch zugegeben werden muss.

2. Verwendung von Trehalose gemäß Anspruch 1, wobei die Trehalose dem Reaktionsgemisch in einer Konzentration von 10% (Masse/Vol) zugegeben wird.

3. Verwendung nach Anspruch 1 oder 2, **dadurch gekennzeichnet, daß** die nativen Proteingemische Zellextrakte, Zelllysate oder Fraktionen aus diesen sind.

4. Verwendung nach einem der Ansprüche 1-3, **dadurch gekennzeichnet, daß** die artifiziellen Proteingemische eine Kombination aus vorgereinigten Einzelenzymen und Kofaktoren sind.

5. Verwendung nach einem der Ansprüche 1-4, **dadurch gekennzeichnet, daß** bei der Überführung der Reaktionsgemische in den lagerstabilen Zustand eine Vakuumtrocknung bei Raumtemperatur in einer handelsüblichen Lyophilisationsanlage in einer Zeitspanne von 3-4 Stunden durchgeführt wird.

6. Verwendung nach Anspruch 5, **dadurch gekennzeichnet, daß** das Reaktionsgemisch vor der Vakuumtrocknung in flüssigem Stickstoff oder gegebenenfalls in einem Trockeneis/Alkoholbad eingefroren wird.

## Claims

1. Use of trehalose in multi-enzymatic reaction mixtures that are converted by means of freeze-drying into a state that is stable in storage, for simultaneously increasing stability in storage and reactivity in such a way that, after freeze-drying, reconstituted reaction mixtures show greater enzymatic activity than fresh reaction mixtures without trehalose, whereby the reaction mixtures contain native or artificial active protein mixtures and the trehalose must be added to the reaction mixture in a concentration of 8-12% (mass/vol) of the reaction mixture.

2. Use of trehalose according to Claim 1, whereby the trehalose is added to the reaction mixture in a concentration of 10% (mass/vol) of the reaction mixture.

3. Use according to Claim 1 or 2, **characterised in that** the native protein mixtures are cell extracts, cell lysates or fractions of these.

4. Use according to one of the Claims 1-3, **characterised in that** the artificial protein mixtures are a combination of prepurified individual enzymes and cofactors.

5. Use according to one of the Claims 1-4, **characterised in that**, when converting the reaction mixture into a state that is stable in storage, vacuum-drying at room temperature is carried out over a period of 3-4 hours in a traditional commercial lyophilisation device.

6. Use according to Claim 5, **characterised in that** the reaction mixture is frozen in liquid nitrogen or optionally in dry ice alcohol bath prior to the vacuum-drying.

## Revendications

1. Recours au tréhalose dans des mélanges réactionnels multienzymatiques, devenus stables à la conservation suite à une action de séchage par congélation, afin d'augmenter parallèlement la stabilité de conservation ainsi que la réactivité, de sorte qu'à l'issue du séchage par congélation les mélanges réactionnels reconstitués présentent une activité enzymatique plus élevée que les mélanges réactionnels « frais », les mélanges réactionnels contenant des mélanges de protéines actifs, natifs ou artificiels, et le tréhalose devant être ajouté au mélange réactionnel selon une concentration de 8-12% (masse/vol.).

2. Recours au tréhalose selon la revendication 1, le tréhalose étant ajouté au mélange réactionnel selon une concentration de 10% (masse/vol.).

3. Recours au tréhalose selon les revendications 1 ou 2, **caractérisé en ce que** les mélanges de protéines natifs sont en fait composés à partir d'extraits de cellules, de lysats de cellules ou de fractions de ces mélanges.

4. Recours au tréhalose selon l'une des revendications 1 à 3, **caractérisé en ce que** les mélanges de protéines artificiels constituent une combinaison d'enzymes uniques préalablement purifiés et de cofacteurs.

5. Recours au tréhalose selon l'une des revendications 1 à 4, **caractérisé en ce qu'**un séchage sous vide est opéré à température ambiante dans un dispositif de lyophilisation courant en respectant un intervalle de 3-4 heures, et ce lors du transfert des mélanges réactionnels dans un état stable à la conservation.

6. Recours au tréhalose selon la revendication 5, **caractérisé en ce que** le mélange réactionnel est congelé dans de l'azote liquide avant le séchage sous vide ou, le cas échéant, en carboglace/dans un bain d'alcool.
